# EUROPEAN PATENT APPLICATION

(11) **EP 4 632 759 A1**
(43) Date of publication of application: **15.10.2025**
(21) Application number: 25169410.5
(22) Date of filing: 09.04.2025
(51) Int. Cl.: G16H 30/40, G16H 40/63

(54) **SYSTEM, METHOD, AND COMPUTER PROGRAM PRODUCT FOR DETECTING CONNECTION OF A MEDICAL DEVICE TO A NEEDLELESS CONNECTOR**

(30) Priority: 09.04.2024 US 202418630427
(71) Applicant: Becton, Dickinson and Company, Franklin Lakes, NJ 07417 (US)
(72) Inventor: ELLERBUSCH, Gary, Nutley, 07110 (US); VERMA, Kaushal Kumar, Bridgewater, 08807-5604 (US)
(74) Representative: dompatent von Kreisler Selting Werner - Partnerschaft von Patent- und Rechtsanwälten mbB

(57) **Abstract**

Systems, methods, and computer program products are provided for detecting connection of a medical device to a needleless connector. An example system includes an image capture device and at least one processor. The image capture device may be configured to capture a set of images of a movable component of a needleless connector. The at least one processor may be configured to receive the set of images of the movable component of the needleless connector; and process the set of images of the movable component of the needleless connector to determine whether a medical device is connected to the needleless connector.

## Description

### CROSS-REFERENCE TO RELATED APPLICATION

The present application claims priority to United States Patent Application No. 18/630,427 entitled "System, Method, and Computer Program Product for Detecting Connection of a Medical Device to a Needleless Connection" filed April 9, 2024, the disclosure of which is hereby incorporated by reference in its entirety.

### BACKGROUND

### Technical Field

This disclosure relates generally to needleless connectors and, in non-limiting embodiments or aspects, to systems, methods, and computer program products for detecting connection of a medical device to a needleless connector.

### Technical Considerations

In clinical use, IV tubing is connected to a patient in order to administer medications. IV tubing may include needleless connectors to which medication-filled medical devices (e.g., syringes, etc.) are connected for medication administration. As use of the Internet of Things (IoT) spreads in the medical field, there may be a need to detect/record when a medical device (e.g., a syringe, etc.) is attached to these needleless connectors.

### SUMMARY

Accordingly, provided are improved systems, methods, and computer program products for detecting connection of a medical device to a needleless connector.

According to non-limiting embodiments or aspects, provided is a system including: an image capture device configured to capture a set of images of a movable component of a needleless connector; and at least one processor configured to: receive the set of images of the movable component of the needleless connector; and process the set of images of the movable component of the needleless connector to determine whether a medical device is connected to the needleless connector.

In some non-limiting embodiments or aspects, the movable component of the needleless connector includes a bellow, and wherein the bellow is configured to compress from an expanded state to a compressed state in response to a connection of the medical device to the needleless connector.

In some non-limiting embodiments or aspects, the needleless connector includes a fluid flow path in a housing between an inlet and an outlet opposite the inlet, and wherein the bellow is included within the housing.

In some non-limiting embodiments or aspects, the image capture device is integrated with the housing of the needleless connector.

In some non-limiting embodiments or aspects, the image capture device is external to the housing of the needleless connector.

In some non-limiting embodiments or aspects, the image capture device includes a complementary metal oxide semiconductor (CMOS) sensor.

In some non-limiting embodiments or aspects, the system further includes: communication circuitry configured to communicate with an external computing device.

In some non-limiting embodiments or aspects, the at least one processor is configured to process the set of images of the movable component of the needleless connector to determine whether the medical device is connected to the needleless connector by: comparing pixels of an image of the set of images to pixels of another image of the set of images to determine a shift in a pixel pattern between the image and the another image; converting a shift in the pixel pattern between the image and the another image to a shift in x-coordinate distance of the movable component or a shift in y-coordinate distance of the movable component; and determining, based on the shift in x-coordinate distance of the movable component or the shift in y-coordinate distance of the movable component, whether the medical device is connected to the needleless connector.

In some non-limiting embodiments or aspects, the medical device includes a syringe.

According to some non-limiting embodiments or aspects, provided is a needleless connector including: a moveable component having a first position when the needleless connector is connected with a medical device and a second position when the needleless connector is not connected with a medical device; an image capture device configured to capture a set of images of the movable component of the needleless connector; communication circuitry configured to communicate with an external computing device; and at least one processor configured to: receive the set of images of the movable component of the needleless connector; process the set of images of the movable component of the needleless connector to determine whether a medical device is connected to the needleless connector.

According to some non-limiting embodiments or aspects, provided is a method including: capturing, with an image capture device, a set of images of a movable component of a needleless connector; receiving, with at least one processor, the set of images of the movable component of the needleless connector; processing, with the at least one processor, the set of images of the movable component of the needleless connector to determine whether the medical device is connected to the needleless connector; and providing, with the at least one processor, an indication of whether the medical device is connected to the needleless connector.

In some non-limiting embodiments or aspects, the movable component of the needleless connector includes a bellow, wherein the bellow is configured to compress from an expanded state to a compressed state in response to a connection of the medical device to the needleless connector, and wherein processing the set of images to determine whether medical device is connected to the needleless connector includes identifying a state of the bellow as shown in the set of images.

In some non-limiting embodiments or aspects, the needleless connector includes a fluid flow path in a housing between an inlet and an outlet opposite the inlet, and wherein the bellow is included within the housing, and wherein capturing, with the image capture device, the set of images of the movable component of the needleless connector includes capturing, with the image capture device, the set of images of the movable component of the needleless connector through a light transmissive portion of the housing.

In some non-limiting embodiments or aspects, capturing, with the image capture device, the set of images of the movable component of the needleless connector includes capturing the set of images based on at least one of the following: visible light, non-visible light, thermal radiation, depth, motion, or any combination thereof.

In some non-limiting embodiments or aspects, the method further includes: communicating, with communication circuitry, the set of images to an external computing device, wherein the external computing devices includes the at least one processor.

In some non-limiting embodiments or aspects, processing the set of images of the movable component of the needleless connector to determine whether the medical device is connected to the needleless connector includes: providing, with the at least one processor, as input to a machine learning model, the set of images; and receiving, with the at least one processor, as output from the machine learning model, a prediction of whether the medical device is connected to the needleless connector.

In some non-limiting embodiments or aspects, providing, with the at least one processor, the indication of whether the medical device is connected to the needleless connector includes: generating, with the at least one processor, a message including at least: (i) the indication of whether the medical device is connected to the needleless connector, and (ii) an identifier of the medical device or of the needleless connector; and communicating, with communication circuitry, the message to an external computing device.

In some non-limiting embodiments or aspects, processing the set of images of the movable component of the needleless connector to determine whether the medical device is connected to the needleless connector includes: comparing, with the at least one processor, pixels of an image of the set of images to pixels of another image of the set of images to determine a shift in a pixel pattern between the image and the another image; converting, with the at least one processor, a shift in the pixel pattern between the image and the another image to a shift in x-coordinate distance of the movable component or a shift in y-coordinate distance of the movable component; and determining, with the at least one processor, based on the shift in x-coordinate distance of the movable component or the shift in y-coordinate distance of the movable component, whether the medical device is connected to the needleless connector.

In some non-limiting embodiments or aspects, processing the set of images of the movable component of the needleless connector to determine whether the medical device is connected to the needleless connector includes: detecting, with the at least one processor, a frequency of one or more colors in one or more images of the set of images; and determining, with the at least one processor, based on the frequency of the one or more colors in the one or more images of the set of images and one or more threshold frequencies, whether the medical device is connected to the needleless connector.

In some non-limiting embodiments or aspects, the medical device includes a syringe that includes a Luer tip, wherein the moveable component has a first position when the Luer tip of the syringe is connected with the needleless connector and a second position when the Luer tip of the syringe is not connected with the needleless connector, and wherein processing the set of images of the movable component of the needleless connector to determine whether the medical device is connected to the needleless connector includes: determining, based on the set of images, whether the moveable component is in the first position or the second position.

According to some non-limiting embodiments or aspects, provided is a computer program product comprising at least one non-transitory computer-readable medium including program instructions that, when executed by at least one processor, cause the at least one processor to: receive a set of images of a movable component of a needleless connector; process the set of images of the movable component of the needleless connector to determine whether a medical device is connected to the needleless connector; and provide an indication of whether the medical device is connected to the needleless connector.

Further non-limiting embodiments or aspects are set forth in the following numbered clauses:
Clause 1: A system comprising: an image capture device configured to capture a set of images of a movable component of a needleless connector; and at least one processor configured to: receive the set of images of the movable component of the needleless connector; and process the set of images of the movable component of the needleless connector to determine whether a medical device is connected to the needleless connector.
Clause 2: The system of clause 1, wherein the movable component of the needleless connector includes a bellow, and wherein the bellow is configured to compress from an expanded state to a compressed state in response to a connection of the medical device to the needleless connector.
Clause 3: The system of clause 1 or clause 2, wherein the needleless connector includes a fluid flow path in a housing between an inlet and an outlet opposite the inlet, and wherein the bellow is included within the housing.
Clause 4: The system of any of clauses 1-3, wherein the image capture device is integrated with the housing of the needleless connector.
Clause 5: The system of any of clauses 1-4, wherein the image capture device is external to the housing of the needleless connector.
Clause 6: The system of any of clauses 1-5, wherein the image capture device includes a complementary metal oxide semiconductor (CMOS) sensor.
Clause 7: The system of any of clauses 1-6, further comprising: communication circuitry configured to communicate with an external computing device.
Clause 8: The system of any of clauses 1-7, wherein the at least one processor is configured to process the set of images of the movable component of the needleless connector to determine whether the medical device is connected to the needleless connector by: comparing pixels of an image of the set of images to pixels of another image of the set of images to determine a shift in a pixel pattern between the image and the another image; converting a shift in the pixel pattern between the image and the another image to a shift in x-coordinate distance of the movable component or a shift in y-coordinate distance of the movable component; and determining, based on the shift in x-coordinate distance of the movable component or the shift in y-coordinate distance of the movable component, whether the medical device is connected to the needleless connector.
Clause 9: The system of any of clauses 1-8, wherein the medical device includes a syringe.
Clause 10. A needleless connector comprising: a moveable component having a first position when the needleless connector is connected with a medical device and a second position when the needleless connector is not connected with a medical device; an image capture device configured to capture a set of images of the movable component of the needleless connector; communication circuitry configured to communicate with an external computing device; and at least one processor configured to: receive the set of images of the movable component of the needleless connector; and process the set of images of the movable component of the needleless connector to determine whether a medical device is connected to the needleless connector.
Clause 11: A method comprising: capturing, with an image capture device, a set of images of a movable component of a needleless connector; receiving, with at least one processor, the set of images of the movable component of the needleless connector; processing, with the at least one processor, the set of images of the movable component of the needleless connector to determine whether the medical device is connected to the needleless connector; and providing, with the at least one processor, an indication of whether the medical device is connected to the needleless connector.
Clause 12: The method of clause 11, wherein the movable component of the needleless connector includes a bellow, wherein the bellow is configured to compress from an expanded state to a compressed state in response to a connection of the medical device to the needleless connector, and wherein processing the set of images to determine whether medical device is connected to the needleless connector includes identifying a state of the bellow as shown in the set of images.
Clause 13. The method of clause 11 or clause 12, wherein the needleless connector includes a fluid flow path in a housing between an inlet and an outlet opposite the inlet, and wherein the bellow is included within the housing, and wherein capturing, with the image capture device, the set of images of the movable component of the needleless connector includes capturing, with the image capture device, the set of images of the movable component of the needleless connector through a light transmissive portion of the housing.
Clause 14. The method of any of clauses 11-13, wherein capturing, with the image capture device, the set of images of the movable component of the needleless connector includes capturing the set of images based on at least one of the following: visible light, non-visible light, thermal radiation, depth, motion, or any combination thereof.
Clause 15: The method of any of clauses 11-14, further comprising: communicating, with communication circuitry, the set of images to an external computing device, wherein the external computing devices includes the at least one processor.
Clause 16. The method of any of clauses 11-15, wherein processing the set of images of the movable component of the needleless connector to determine whether the medical device is connected to the needleless connector includes: providing, with the at least one processor, as input to a machine learning model, the set of images; and receiving, with the at least one processor, as output from the machine learning model, a prediction of whether the medical device is connected to the needleless connector.
Clause 17. The method of any of clauses 11-16, wherein providing, with the at least one processor, the indication of whether the medical device is connected to the needleless connector includes: generating, with the at least one processor, a message including at least: (i) the indication of whether the medical device is connected to the needleless connector, and (ii) an identifier of the medical device or of the needleless connector; and communicating, with communication circuitry, the message to an external computing device.
Clause 18: The method of any of clauses 11-17, wherein processing the set of images of the movable component of the needleless connector to determine whether the medical device is connected to the needleless connector includes: comparing, with the at least one processor, pixels of an image of the set of images to pixels of another image of the set of images to determine a shift in a pixel pattern between the image and the another image; converting, with the at least one processor, a shift in the pixel pattern between the image and the another image to a shift in x-coordinate distance of the movable component or a shift in y-coordinate distance of the movable component; and determining, with the at least one processor, based on the shift in x-coordinate distance of the movable component or the shift in y-coordinate distance of the movable component, whether the medical device is connected to the needleless connector.
Clause 19. The method of any of clauses 11-18, wherein processing the set of images of the movable component of the needleless connector to determine whether the medical device is connected to the needleless connector includes: detecting, with the at least one processor, a frequency of one or more colors in one or more images of the set of images; and determining, with the at least one processor, based on the frequency of the one or more colors in the one or more images of the set of images and one or more threshold frequencies, whether the medical device is connected to the needleless connector.
Clause 20. The method of any of clauses 11-19, wherein the medical device includes a syringe that includes a Luer tip, wherein the moveable component has a first position when the Luer tip of the syringe is connected with the needleless connector and a second position when the Luer tip of the syringe is not connected with the needleless connector, and wherein processing the set of images of the movable component of the needleless connector to determine whether the medical device is connected to the needleless connector includes: determining, based on the set of images, whether the moveable component is in the first position or the second position.
Clause 21. A computer program product comprising at least one non-transitory computer-readable medium including program instructions that, when executed by at least one processor, cause the at least one processor to: receive a set of images of a movable component of a needleless connector; process the set of images of the movable component of the needleless connector to determine whether a medical device is connected to the needleless connector; and provide an indication of whether the medical device is connected to the needleless connector.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosed subject matter.

### BRIEF DESCRIPTION OF THE DRAWINGS

Additional advantages and details are explained in greater detail below with reference to the non-limiting, exemplary embodiments that are illustrated in the accompanying schematic figures, in which:
FIG. 1 is a schematic diagram of a system for detecting connection of a medical device to a needleless connector, according to some non-limiting embodiments or aspects;
FIG. 2 is a schematic diagram of example components of one or more devices of FIG. 1, according to some non-limiting embodiments or aspects;
FIG. 3A is a perspective view of an implementation of a needleless connector, according to some non-limiting embodiments or aspects;
FIG. 3B is a perspective view of an implementation of a moveable component of a needleless connector, according to some non-limiting embodiments or aspects;
FIG. 4 is a flow diagram of a method for detecting connection of a medical device to a needleless connector, according to some non-limiting embodiments or aspects; and
FIGS. 5A and 5B are perspective views of an implementation of a needleless connector, according to some non-limiting embodiments or aspects.

### DETAILED DESCRIPTION

For purposes of the description hereinafter, the terms "end," "upper," "lower," "right," "left," "vertical," "horizontal," "top," "bottom," "lateral," "longitudinal," and derivatives thereof shall relate to the embodiments as they are oriented in the drawing figures. However, it is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects of the disclosed subject matter. Hence, specific dimensions and other physical characteristics related to the embodiments or aspects disclosed herein are not to be considered as limiting.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

No aspect, component, element, structure, act, step, function, instruction, and/or the like used herein should be construed as critical or essential unless explicitly described as such. Also, as used herein, the articles "a" and "an" are intended to include one or more items and may be used interchangeably with "one or more" and "at least one." Furthermore, as used herein, the term "set" is intended to include one or more items (e.g., related items, unrelated items, a combination of related and unrelated items, and/or the like) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise. In addition, reference to an action being "based on" a condition may refer to the action being "in response to" the condition. For example, the phrases "based on" and "in response to" may, in some non-limiting embodiments or aspects, refer to a condition for automatically triggering an action (e.g., a specific operation of an electronic device, such as a computing device, a processor, and/or the like).

As used herein, the term "communication" may refer to the reception, receipt, transmission, transfer, provision, and/or the like of data (e.g., information, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection (e.g., a direct communication connection, an indirect communication connection, and/or the like) that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices configured to process data. A computing device may, in some examples, include the necessary components to receive, process, and output data, such as a processor, a display, a memory, an input device, a network interface, and/or the like. A computing device may be a mobile device. As an example, a mobile device may include a cellular phone (e.g., a smartphone or standard cellular phone), a portable computer, a wearable device (e.g., watches, glasses, lenses, clothing, and/or the like), a personal digital assistant (PDA), and/or other like devices. A computing device may also be a desktop computer or other form of non-mobile computer.

As used herein, the term "server" may refer to or include one or more computing devices that are operated by or facilitate communication and processing for multiple parties in a network environment, such as the Internet, although it will be appreciated that communication may be facilitated over one or more public or private network environments and that various other arrangements are possible. Further, multiple computing devices (e.g., servers, point-of-sale (POS) devices, mobile devices, etc.) directly or indirectly communicating in the network environment may constitute a "system."

As used herein, the term "system" may refer to one or more computing devices or combinations of computing devices (e.g., processors, servers, client devices, software applications, components of such, and/or the like). Reference to "a device," "a server," "a processor," and/or the like, as used herein, may refer to a previously-recited device, server, or processor that is recited as performing a previous step or function, a different device, server, or processor, and/or a combination of devices, servers, and/or processors. For example, as used in the specification and the claims, a first device, a first server, or a first processor that is recited as performing a first step or a first function may refer to the same or different device, server, or processor recited as performing a second step or a second function.

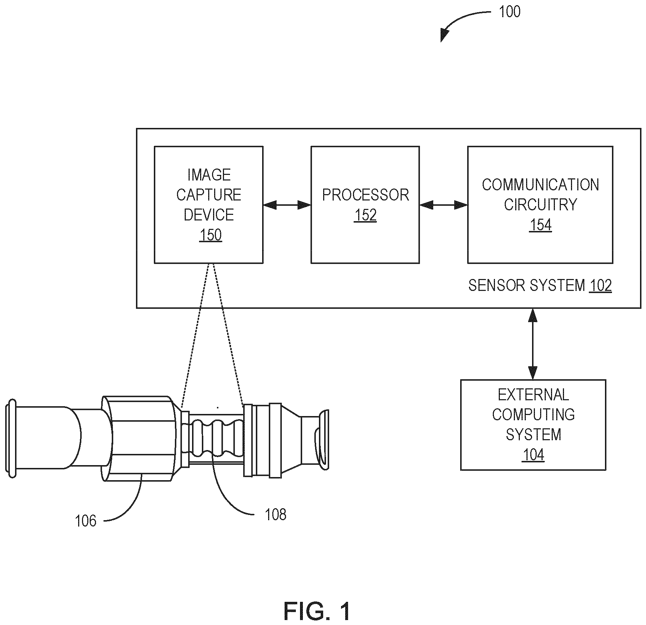

Referring now to FIG. 1, shown is a schematic diagram of a system for detecting connection of a medical device to a needleless connector, according to some non-limiting embodiments or aspects. As shown in FIG. 1, system 100 may include sensor system 102, needleless connector 106, and/or external computing system 106. Systems and/or devices of system 100 can interconnect via wired connections, wireless connections, or a combination of wired and wireless connections.

Sensor system 102 may include one or more devices capable of receiving information and/or data from external computing device 104 and/or communicating information and/or data to external computing device 104. For example, sensor system 102 may include one or more computing systems including one or more processors (e.g., one or more computing devices, one or more mobile computing devices, one or more digital signal processors (DSPs), etc.).

External computing system 104 may include one or more devices capable of receiving information and/or data from sensor system 102 and/or communicating information and/or data to sensor system 102. For example, external computing system 104 may include one or more computing systems including one or more processors (e.g., one or more computing devices, one or more mobile computing devices, one or more servers, etc.). In some non-limiting embodiments or aspects, external computing system 104 includes a nurse station in a hospital, a hospital information system (HIS), an electronic medical records (EMR) system, a radiology information system (RIS), a picture archiving and communication system (PACS), a laboratory information system (LIS), a smart phone, a tablet computer, any combination thereof, and/or the like.

Sensor system 102 may include image capture device 150, processor 152, and/or communication circuitry 154.

Image capture device 150 may be configured to capture a set of images (e.g., a single image, a plurality of images, a series of images, a sequence of images, etc.) of movable component 108 of needleless connector 106. For example, a field-of-view of image capture device 150 may be configured to include movable component 108 of needleless connector 106. Image capture device 150 may include a complementary metal oxide semiconductor (CMOS) sensor, a charge coupled device (CCD), a camera, or the like. Image capture device 150 may be configured to provide the set of images of movable component 108 of needleless connector 106 to processor 152. The image capture device 150 may be configured to capture visible light, non-visible light (e.g., infrared), thermal data (e.g., thermal camera), depth (e.g., depth sensing camera), or motion detecting camera. For example, in response to an outlet end of a medical device (e.g., an outlet tip of a syringe, such as a luer tip, etc.) being connected to needleless connector (e.g., being inserted into or received within needleless connector 106, etc.), the outlet end of the medical device may contact movable component 108 to cause a movement of movable component 108 that is captured in the set of images.

In some non-limiting embodiments or aspects, image capture device 150 may include a lens configured to increase a clarity of the set of images captured and/or to adjust an optical distance between image capture device 150 and movable component 108. In some non-limiting embodiments or aspects, image capture device 150 and/or a field-of-view thereof may be held or fixed at a constant position relative to movable component 108 (e.g., by needleless connector 106 or another device connected thereto, etc.). In some non-limiting embodiments or aspects, image capture device 150 may be movable relative to movable component 108 and/or needleless connector 106 (e.g., by a user moving a tablet computer or other device including image capture device 150, etc.).

Processor 152 may be configured to receive the set of images of movable component 108 of needleless connector 106. Processor 152 may be configured to process the set of images of movable component 108 of needleless connector 106 to determine whether a medical device (e.g., a syringe, an IV bag, an IV line, another needleless connector, etc.) is connected to needleless connector 106. For example, processor 152 may be configured to compare pixels of an image of the set of images to pixels of another image of the set of images (e.g., on a pixel-by-pixel basis, etc.) to determine a shift in a pixel pattern between the image and the another image. Processor 152 may convert a shift in the pixel pattern between the image and the another image to a shift in x-coordinate distance or a shift in y-coordinate distance. Processor 152 may determine, based on the shift in x-coordinate distance of movable component 108 or the shift in y-coordinate distance of movable component 108, whether the medical device is connected to needleless connector 106. For example, processor 152 may determine a first change (e.g., a positive change, etc.) in x-coordinate distance or y-coordinate distance of movable component 108 as a medical device (e.g., a syringe, etc.) connected or being connected to needleless connector 106, and/or processor 152 may determine a second change (e.g., a negative change, etc.) in x-coordinate distance or y-coordinate distance as a medical device (e.g., a syringe, etc.) disconnected or being disconnected from needleless connector 106. However, non-limiting embodiments or aspects are not limited thereto, and processor 152 may use other image processing techniques and/or object recognition techniques to process the set of images of movable component 108 of needleless connector 106 to determine movement of the movable component and, based thereon, whether a medical device (e.g., a syringe, etc.) is connected to needleless connector 106.

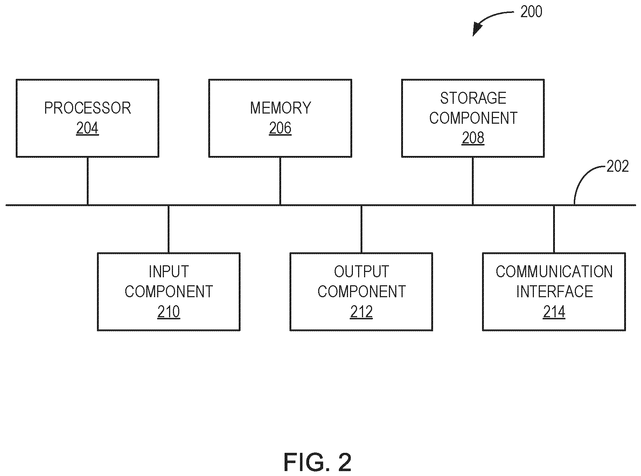

Processor 152 may include a digital signal processor (DSP), a microcontroller, processor 204 of FIG. 2, any combination thereof, and/or the like. In some non-limiting embodiments or aspects, image capture device 150 and processor 152 may be implemented as a CMOS sensor with integrated DSP.

Communication circuitry 154 (e.g., communication interface 214, etc.) may be configured to communicate with external computing system 104 (e.g., with an external computing device, etc.). For example, communication circuitry 154 may be configured to communicate with external computing system 104 via a one or more wired connections, one or more wireless connections, or a combination of one or more wired and one or more wireless connections. As example, communication circuitry 154 may establish a short range wireless communication connection (e.g., an NFC communication connection, an RFID communication connection, a Bluetooth^{®} communication connection, etc.) with external computing system 104.

The number and arrangement of systems and devices shown in FIG. 1 are provided as an example. There may be additional systems or devices, fewer systems or devices, different systems or devices, or differently arranged systems or devices than those shown in FIG. 1. Furthermore, two or more systems or devices shown in FIG. 1 may be implemented within a single system or device, or a single system or device shown in FIG. 1 may be implemented as multiple, distributed systems or devices. Additionally or alternatively, a set of systems (e.g., one or more systems) or a set of devices (e.g., one or more devices) of system 100 may perform one or more functions described as being performed by another set of systems or another set of devices of system 100.

Referring now to FIG. 2, shown is a diagram of example components of a device 200 according to non-limiting embodiments. Device 200 may correspond to sensor system 102, needleless connector 106, and/or external computing system 104 in FIG. 1, as an example. In some non-limiting embodiments, such systems or devices may include at least one device 200 and/or at least one component of device 200. The number and arrangement of components shown are provided as an example. In some non-limiting embodiments, device 200 may include additional components, fewer components, different components, or differently arranged components than those shown. Additionally, or alternatively, a set of components (e.g., one or more components) of device 200 may perform one or more functions described as being performed by another set of components of device 200.

As shown in FIG. 2, device 200 may include a bus 202, a processor 204, memory 206, a storage component 208, an input component 210, an output component 212, and a communication interface 214. Bus 202 may include a component that permits communication among the components of device 200. In some non-limiting embodiments, processor 204 may be implemented in hardware, firmware, or a combination of hardware and software. For example, processor 204 may include a processor (e.g., a central processing unit (CPU), a graphics processing unit (GPU), an accelerated processing unit (APU), etc.), a microprocessor, a digital signal processor (DSP), or any processing component (e.g., a field-programmable gate array (FPGA), an application-specific integrated circuit (ASIC), etc.) that can be programmed to perform a function. Memory 206 may include random access memory (RAM), read only memory (ROM), or another type of dynamic or static storage device (e.g., flash memory, magnetic memory, optical memory, etc.) that stores information and/or instructions for use by processor 204.

With continued reference to FIG. 2, storage component 208 may store information and/or software related to the operation and use of device 200. For example, storage component 208 may include a hard disk (e.g., a magnetic disk, an optical disk, a magneto-optic disk, a solid-state disk, etc.) or another type of computer-readable medium. Input component 210 may include a component that permits device 200 to receive information, such as via user input (e.g., a touch screen display, a keyboard, a keypad, a mouse, a button, a switch, a microphone, etc.). Additionally, or alternatively, input component 210 may include a sensor for sensing information (e.g., a global positioning system (GPS) component, an accelerometer, a gyroscope, an actuator, etc.). Output component 212 may include a component that provides output information from device 200 (e.g., a display, a speaker, one or more light-emitting diodes (LEDs), etc.). Communication interface 214 may include a transceiver-like component (e.g., a transceiver, a separate receiver and transmitter, etc.) that enables device 200 to communicate with other devices, such as via a wired connection, a wireless connection, or a combination of wired and wireless connections. Communication interface 214 may permit device 200 to receive information from another device or provide information to another device. For example, communication interface 214 may include an Ethernet interface, an optical interface, a coaxial interface, an infrared interface, a radio frequency (RF) interface, a universal serial bus (USB) interface, a Wi-Fi^{®} interface, a cellular network interface, or the like.

Device 200 may perform one or more processes described herein. Device 200 may perform these processes based on processor 204 executing software instructions stored by a computer-readable medium, such as memory 206 or storage component 208. A computer-readable medium may include any non-transitory memory device. A memory device includes memory space located inside of a single physical storage device or memory space spread across multiple physical storage devices. Software instructions may be read into memory 206 and/or storage component 208 from another computer-readable medium or from another device via communication interface 214. When executed, software instructions stored in memory 206 or storage component 208 may cause processor 204 to perform one or more processes described herein. Additionally, or alternatively, hardwired circuitry may be used in place of or in combination with software instructions to perform one or more processes described herein. Thus, embodiments described herein are not limited to any specific combination of hardware circuitry and software. The term "configured to," as used herein, may refer to a specific arrangement of software, device(s), or hardware for performing or enabling one or more of the innovative functions (e.g., actions, processes, steps of a process, or the like) described herein. For example, "a processor configured to" may refer to a processor that executes specific software instructions (e.g., program code) that cause the processor to perform one or more functions related to needle free connection detection.

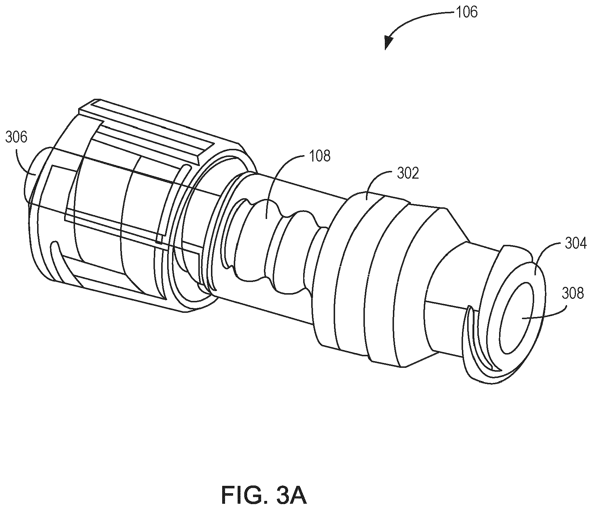

Referring again to FIG. 1, and also to FIG. 3A, which shows a perspective view of an implementation of needleless connector 106, according to some non-limiting embodiments or aspects, needleless connector 106 may include a fluid flow path in a housing 302 between inlet 304 and outlet 306 opposite inlet 304. Inlet 304 may be fluidically sealed by displaceable septum 308 configured to be displaced to open or connect inlet 304 to the fluid flow path in response to connection of needleless connector 106 to a medical device (e.g., a syringe or other fluidic device or container). For example, needleless connector 106 may include the BD SmartSite^{®}, BD Q-Syte^{™}, BD MaxZero^{™}, or BD MaxPlus^{™} needle free connectors, or similar connector devices. However, non-limiting embodiments or aspects are not limited thereto, and needleless connector 106 may include any needleless connector for use in fluid administration that includes any movable component configured to move in response to a connection of a medical device to needleless connector 106. For example, needleless connector 106 may include a moveable component having a first position when needleless connector 106 is connected with a medical device and a second position when needleless 106 is not connected with a medical device. As an example, processor 152 may be configured to process the set of images of the movable component of needleless connector 106 to determine whether the medical device is connected to the needleless connector by determining, based on the set of images, whether the moveable component is in the first position or the second position. Needleless connector 106 may include a port, a manifold, a stopcock, an open connector, a Luer connector, or any other connector that does not rely on (but may or may not include) a needle to form a connection with a device or a patient.

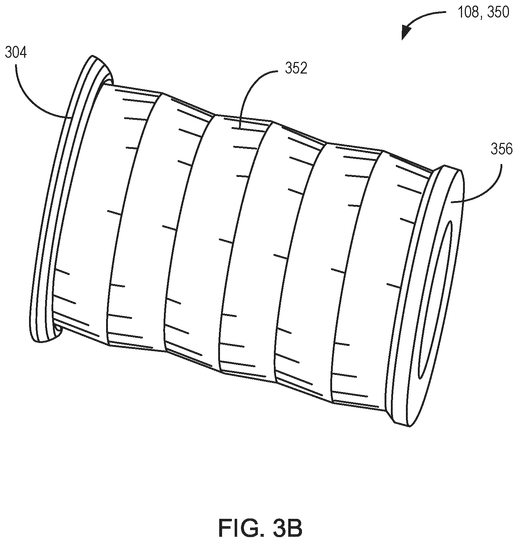

Still referring to FIGS. 1 and 3A, and referring also to FIG. 3B, which shows a perspective view of a of an implementation of moveable component 108 of needleless connector 106, according to some non-limiting embodiments or aspects, moveable component 108 of needleless connector 106 may include a bellow 350. Bellow 350 may include a compressible or deformable body 352 that is cylindrical in shape. Compressible or deformable body 352 may include an accordion shape with multiple annular folds. Bellow 350 may include inlet lip 354 and outlet lip 356. Bellow 350 may be positioned within housing 302 of needleless connector 106.

Bellow 350 may be is configured to compress or deform from an expanded state to a compressed or deformed state in response to a connection of a medical device to needleless connector 106. For example, bellow 350 may be configured to compress or deform within housing 302 of needleless connector 106 in response to a connection of a medical device (e.g., a syringe or other fluidic device or container) to needleless connector 106. As an example, in response to an outlet end of a medical device (e.g., an outlet tip of a syringe, such as a luer tip, etc.) being inserted into or received within inlet 304 of needleless connector 106 and/or displacing displaceable septum 308 to open or connect inlet 304 to the fluid flow path, the outlet end of the medical device may contact inlet lip 354 of bellow 350 to compress or deform bellow 350 in a direction away from inlet 304 toward outlet 306.

Bellow 350 may be configured to expand from the compressed or deformed state to the expanded state in response to a disconnection of the medical device from needleless connector 106. For example, bellow 350 may be configured to decompress or reform within housing 302 of needleless connector 106 in response to a disconnection of a medical device (e.g., a syringe, etc.) to needleless connector 106. As an example, in response to the outlet end of the medical device (e.g., a Luer tip of syringe, etc.) being removed from inlet 304 of needleless connector 106 or closing displaceable septum 308 to close or disconnect inlet 304 to the fluid flow path, the outlet end of the medical device may be removed from contact with inlet lip 354 of bellow 350 to allow or enable bellow 350 to decompress or reform in a direction toward inlet 304 away from outlet 306. In such an example, processing the set of images to determine whether medical device is connected to the needleless connector may include identifying a state of the bellow as shown in the set of images.

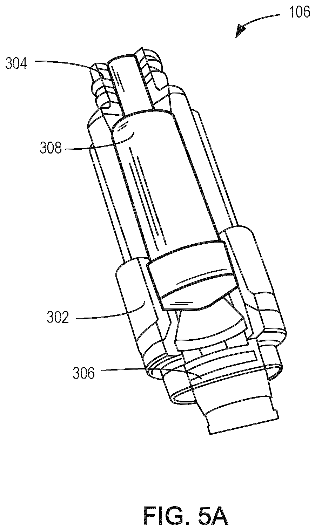

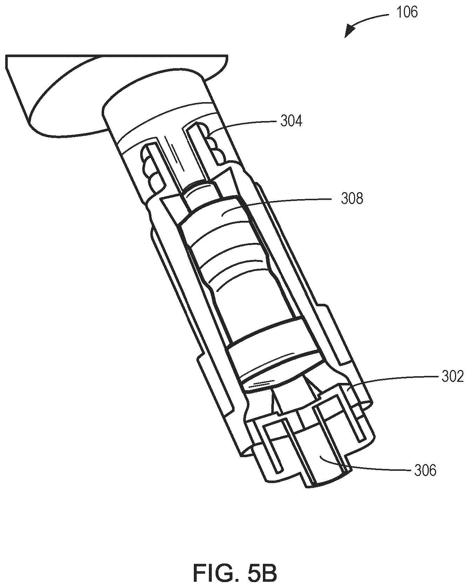

Referring now to FIGS. 5A and 5B, in some implementations, displaceable septum 308 or another element or component connected thereto may be configured to be displaced within housing 302 from an expanded state to a displaced state in response to a connection of a medical device to needleless connector 106. For example, displaceable septum 308 may be configured to be displaced within housing 302 of needleless connector 106 in response to a connection of a medical device (e.g., a syringe or other fluidic device or container) to needleless connector 106. As an example, in response to an outlet end of a medical device (e.g., an outlet tip of a syringe, such as a luer tip, etc.) being inserted into or received within inlet 304 of needleless connector to open or connect inlet 304 to the fluid flow path, the outlet end of the medical device may contact displaceable septum 308 to push or displace displaceable septum 308 and/or another element or component connected thereto in a direction away from inlet 304 toward outlet 306.

Displaceable septum 308 and/or the another component or element connected thereto may be configured to move or expand from the displaced state to the expanded state in response to a disconnection of the medical device from needleless connector 106. For example, displaceable septum 308 may be configured to move or reform within housing 302 of needleless connector 106 in response to a disconnection of a medical device (e.g., a syringe, etc.) to needleless connector 106. As an example, in response to the outlet end of the medical device (e.g., a Luer tip of syringe, etc.) being removed from inlet 304 of needleless connector 106 the outlet end of the medical device may be removed from contact displaceable septum 308 to allow or enable displaceable septum 308 to move in a direction toward inlet 304 away from outlet 306. In such an example, processing the set of images to determine whether medical device is connected to the needleless connector may include identifying a state of displaceable septum 308 and/or the another component connected thereto as shown in the set of images.

In this way, non-limiting embodiments or aspects of the present disclosure may provide a non-contact technique to determine connection (and/or disconnection) of a medical device (e.g., a syringe or other fluid device or container) to needleless connector 106 without being affected by cleaning of needleless connector 106 and/or attachment thereto of a disinfectant cap or other medical device without an outlet tip configured to be received within inlet 304 of needleless connector 106. For example, when needleless connector 106 is cleaned with a swab or a disinfectant cap is attached, bellow 350 remains unchanged, and processor 152 output does not change, thereby enabling sensor sensitivity only to an actual syringe attachment.

In some non-limiting embodiments or aspects, one or more components of sensor system 102 (e.g., image capture device 150, processor 152, communication circuitry 154, etc.) may be included within housing 302 of needleless connector 106. For example, image capture device 150 may be located within or integrated with housing 302 of needleless connector 106 and with a field-of-view including movable component 108. In some non-limiting embodiments or aspects, one or more components of sensor system 102 (e.g., image capture device 150, processor 152, communication circuitry 154, etc.) may be external to housing 302 of needleless connector 106. For example, housing 302 of needleless connector 106 may include a window or light transmissive portion through which image capture device 150 is provided a field-of-view of moveable component 108. As an example, needleless connector 106 may include a fluid injection port of a flow sensor of a flow sensor system including the flow sensor and a base as disclosed by U.S. Patent Application No. 2021/0231471, the disclosure of which is hereby incorporated by reference in its entirety, and/or one or more components of sensor system 102 (e.g., image capture device 150, processor 152, communication circuitry 154, etc.) may be included in or integrated with a housing of the flow sensor and/or the base of the flow sensor system (e.g., proximate the fluid injection port, etc.).

In some implementations, the sensor system 102 may be configured to broadcast a message indicating connection with a syringe or other fluidic container. This message may be broadcast using a standardized communication protocol and standardized message format. In this way, a device, such as the flow sensor system in U.S. Patent Application No. 2021/0231471, can listen for the message and, upon detecting the message indicating a connection, adjust operation. For example, the flow sensor system may include a wireless tag reader to detect a tag attached to the syringe connected to the needleless connector 106. It is not resource efficient to continually activate the wireless tag reader. Instead, the disclosed features may be implemented to provide the connected indication message to the flow sensor system, and, upon receipt, the flow sensor system 102 can activate the wireless tag reader (e.g., near field communication device, RFID device, or the like). In part due to the analysis of an internal physical element of the connector (e.g., the bellows, etc.), the described features also distinguish between connection of the needleless connection with a fluid source (e.g., syringe) and a disinfecting or sterile connector cap. The cap attaches to the needleless connector, but does not cause the bellows to deform in the same manner as when the needleless connector is connected with a syringe.

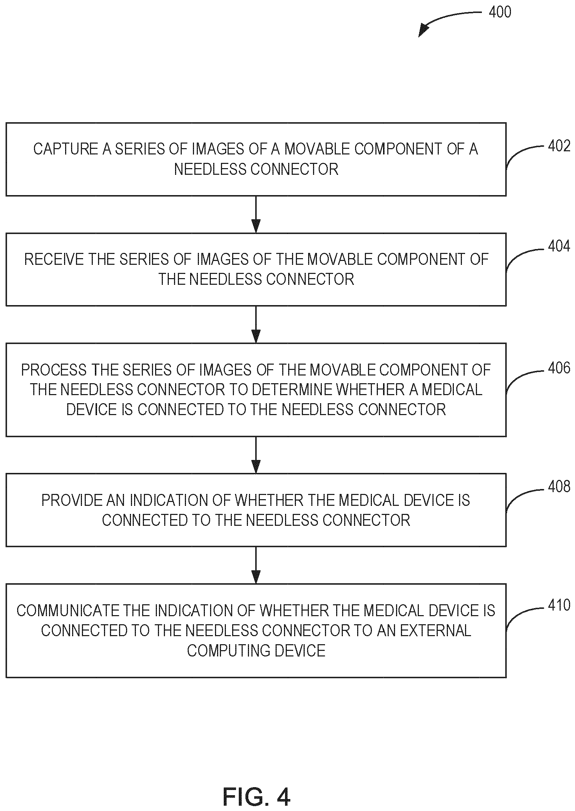

Referring now to FIG. 4, shown is a flow diagram for a method 400 for detecting connection of a medical device to a needleless connector, according to some non-limiting embodiments or aspects. The steps shown in FIG. 4 are for example purposes only. It will be appreciated that additional, fewer, different, or a different order of steps may be used in some non-limiting embodiments or aspects. In some non-limiting embodiments or aspects, a step may be automatically performed in response to performance or completion of a prior step.

As shown in FIG. 4, at step 402, method 400 includes capturing a set of images of a movable component of a needleless connector. For example, image capture device 150 may capture a set of images of movable component 108 of needleless connector 106. The images may be captured as individual images or extracted images from a video. As an example, image capture device 150 may capture the set of images of movable component 108 of needleless connector 106 based on or using on at least one of the following: visible light, non-visible light, thermal radiation, depth, motion, or any combination thereof. For example, image capture device 150 may capture the set of images of movable component 108 of needleless connector 106 through a light transmissive portion of housing 302.

As shown in FIG. 4, at step 404, method 400 includes receiving the set of images of the movable component of the needleless connector. For example, processor 152 may receive the set of images of movable component 108 of needleless connector 106.

As shown in FIG. 4, at step 406, method 400 includes processing the set of images of the movable component of the needleless connector to determine whether a medical device is connected to the needleless connector. For example, processor 152 may process the set of images of movable component 108 of needleless connector 106 to determine whether a medical device is connected to needleless connector 106. As an example, processor 152 may process the set of images of movable component 108 of needleless connector 106 to determine whether the medical device is connected to needleless connector 106 by: comparing pixels of an image of the set of images to pixels of another image of the set of images to determine a shift in a pixel pattern between the image and the another image; converting a shift in the pixel pattern between the image and the another image to a shift in x-coordinate distance of the movable component or a shift in y-coordinate distance of the movable component; and determining, based on the shift in x-coordinate distance of movable component 108 or the shift in y-coordinate distance of movable component 108, whether the medical device is connected to the needleless connector 106.

In some implementations, the processing of the images may include providing one or more images to a model, such as an image classifier model trained using machine learning or other artificial intelligence techniques to accept one or more images as input(s) and provide a predicted connection status as an output. For example, processor 152 may process the set of images of movable component 108 of needleless connector 106 to determine whether a medical device is connected to needleless connector 106 by providing, as input to a machine learning model, the set of images; and receiving, as output from the machine learning model, a prediction of whether the medical device is connected to needleless connector 106.

In some implementations, the image processing may include analyzing colors appearing in the image. Based on frequency of certain colors, the state of the needleless connector 106 may be determined. For example, processor 152 may process the set of images of movable component 108 of needleless connector 106 to determine whether the medical device is connected to needleless connector 106 by detecting a frequency or intensity of one or more colors in one or more images of the set of images; and determine, based on the frequency or intensity of the one or more colors in the one or more images of the set of images and one or more threshold frequencies or intensities, whether the medical device is connected to needleless connector 106. As an example, when the bellows of a needleless connector 106 are compressed, an image of the needleless connector 106 may include more darker shades of blue than when in a disconnected state. The analysis may include identifying metric of the frequency and comparing that metric to a threshold. The analysis may include comparing counts, frequencies, or ratios of certain colors in a first image to that of a second image. If there is a difference or a difference corresponding to a significance threshold, a connection state change may be detected.

As shown in FIG. 4, at step 408, method 400 includes providing an indication of whether the medical device is connected to the needleless connector. For example, processor 152 may generate or provide an indication of whether the medical device is connected to needleless connector 106. As an example, processor 152 may generate a message including at least: (i) the indication of whether the medical device is connected to the needleless connector, and (ii) an identifier of the medical device or of the needleless connector. The indication may be a human perceivable output indicating the connection state. In some implementations, providing the indication may include storing a value in a location of a storage device for subsequent retrieval, transmitting a value directly to the recipient via at least one wired or wireless communication medium, transmitting or storing a reference to a value, and the like. The providing at step 408 may additionally or alternatively include encoding, decoding, encrypting, decrypting, validating, verifying, and the like via a hardware element.

In some non-limiting embodiments or aspects, processor 152 may generate or provide the indication of whether the medical device is connected to needleless connector 106 and/or the identifier of the medical device or of needleless connector 106 in association with patient data associated with a patient, procedure data associated with a patient procedure associated with the patient, caregiver data associated with a caregiver (e.g., a nurse, a doctor, etc.), any combination thereof, or the like. Patient data associated with a patient may include a patient identifier associated with the patient (e.g., a unique patient identifier, etc.), patient demographics (e.g., a name, an age, a sex, a weight, a height, a birthdate, an address, etc.), a list of medication allergies associated with the patient, a list of medication doses delivered, being delivered, and/or pending for delivery to the patient, any combination thereof, or the like. Procedure data may include a procedure identifier associated with the procedure (e.g., a unique procedure identifier, etc.), one or more medical devices associated with the procedure, a name of the procedure, a state of the procedure (e.g., scheduled for a future date and time, currently being performed, previously performed a previous date and time, etc.), a caregiver associated with the procedure, a patient associated with the procedure, any combination thereof, or the like. Caregiver data may include a caregiver identifier associated with the caregiver (e.g., a unique caregiver identifier, etc.), a name of the caregiver, any combination thereof, or the like.

As shown in FIG. 4, at step 410, method 400 includes communicating the indication of whether the medical device is connected to the needleless connector an external computing device. For example, communication circuitry 154 may communicate the indication of whether the medical device is connected to needleless connector 106 to external computing system 10. The communication may include a message that can associate the indication of connection status with a patient or specific medical device to allow storage of the connection status in an electronic medical records system or patient data management system.

Although embodiments have been described in detail for the purpose of illustration, it is to be understood that such detail is solely for that purpose and that the disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment or aspect can be combined with one or more features of any other embodiment or aspect.

Aspects described include artificial intelligence or other operations whereby the system processes inputs and generates outputs with apparent intelligence. The artificial intelligence may be implemented in whole or in part by a model. A model may be implemented as a machine learning model. The learning may be supervised, unsupervised, reinforced, or a hybrid learning whereby multiple learning techniques are employed to generate the model. The learning may be performed as part of training. Training the model may include obtaining a set of training data and adjusting characteristics of the model to obtain a desired model output. For example, three characteristics may be associated with a desired item location. **In** such instance, the training may include receiving the three characteristics as inputs to the model and adjusting the characteristics of the model such that for each set of three characteristics, the output device state matches the desired device state associated with the historical data.

**In** some implementations, the training may be dynamic. For example, the system may update the model using a set of events. The detectable properties from the events may be used to adjust the model.

The model may be an equation, artificial neural network, recurrent neural network, convolutional neural network, decision tree, or other machine-readable artificial intelligence structure. The characteristics of the structure available for adjusting during training may vary based on the model selected. For example, if a neural network is the selected model, characteristics may include input elements, network layers, node density, node activation thresholds, weights between nodes, input or output value weights, or the like. If the model is implemented as an equation (e.g., regression), the characteristics may include weights for the input parameters, thresholds, or limits for evaluating an output value, or criterion for selecting from a set of equations.

Once a model is trained, retraining may be included to refine or update the model to reflect additional data or specific operational conditions. The retraining may be based on one or more signals detected by a device described herein or as part of a method described herein. Upon detection of the designated signals, the system may activate a training process to adjust the model as described.

Further examples of machine learning and modeling features which may be included in the embodiments discussed above are described in "A survey of machine learning for big data processing" by Qiu et al. in EURASIP Journal on Advances in Signal Processing (2016) which is hereby incorporated by reference in its entirety.

## Claims

1. A system comprising:
an image capture device configured to capture a set of images of a movable component of a needleless connector; and
at least one processor configured to:
receive the set of images of the movable component of the needleless connector; and
process the set of images of the movable component of the needleless connector to determine whether a medical device is connected to the needleless connector.

2. The system of claim 1, wherein the movable component of the needleless connector includes a bellow, and wherein the bellow is configured to compress from an expanded state to a compressed state in response to a connection of the medical device to the needleless connector.

3. The system of claim 2, wherein the needleless connector includes a fluid flow path in a housing between an inlet and an outlet opposite the inlet, and wherein the bellow is included within the housing.

4. The system of claim 3, wherein the image capture device is integrated with the housing of the needleless connector.

5. The system of claim 3, wherein the image capture device is external to the housing of the needleless connector.

6. The system of claim 1, wherein the image capture device includes a complementary metal oxide semiconductor (CMOS) sensor.

7. The system of claim 1, further comprising:
communication circuitry configured to communicate with an external computing device.

8. The system of claim 1, wherein the at least one processor is configured to process the set of images of the movable component of the needleless connector to determine whether the medical device is connected to the needleless connector by:
comparing pixels of an image of the set of images to pixels of another image of the set of images to determine a shift in a pixel pattern between the image and the another image;
converting a shift in the pixel pattern between the image and the another image to a shift in x-coordinate distance of the movable component or a shift in y-coordinate distance of the movable component; and
determining, based on the shift in x-coordinate distance of the movable component or the shift in y-coordinate distance of the movable component, whether the medical device is connected to the needleless connector.

9. The system of claim 1, wherein the medical device includes a syringe.

10. A needleless connector comprising:
a moveable component having a first position when the needleless connector is connected with a medical device and a second position when the needleless connector is not connected with a medical device;
an image capture device configured to capture a set of images of the movable component of the needleless connector;
communication circuitry configured to communicate with an external computing device; and
at least one processor configured to:
receive the set of images of the movable component of the needleless connector; and
process the set of images of the movable component of the needleless connector to determine whether a medical device is connected to the needleless connector.

11. A method comprising:
capturing, with an image capture device, a set of images of a movable component of a needleless connector;
receiving, with at least one processor, the set of images of the movable component of the needleless connector;
processing, with the at least one processor, the set of images of the movable component of the needleless connector to determine whether the medical device is connected to the needleless connector; and
providing, with the at least one processor, an indication of whether the medical device is connected to the needleless connector.

12. The method of claim 11, wherein the movable component of the needleless connector includes a bellow,
wherein the bellow is configured to compress from an expanded state to a compressed state in response to a connection of the medical device to the needleless connector, and
wherein processing the set of images to determine whether medical device is connected to the needleless connector includes identifying a state of the bellow as shown in the set of images.

13. The method of claim 12, wherein the needleless connector includes a fluid flow path in a housing between an inlet and an outlet opposite the inlet, and wherein the bellow is included within the housing, and wherein capturing, with the image capture device, the set of images of the movable component of the needleless connector includes capturing, with the image capture device, the set of images of the movable component of the needleless connector through a light transmissive portion of the housing.

14. The method of claim 11, wherein capturing, with the image capture device, the set of images of the movable component of the needleless connector includes capturing the set of images based on at least one of the following: visible light, non-visible light, thermal radiation, depth, motion, or any combination thereof.

15. The method of claim 11, further comprising:
communicating, with communication circuitry, the set of images to an external computing device, wherein the external computing devices includes the at least one processor.

16. The method of claim 11, wherein processing the set of images of the movable component of the needleless connector to determine whether the medical device is connected to the needleless connector includes:
providing, with the at least one processor, as input to a machine learning model, the set of images; and
receiving, with the at least one processor, as output from the machine learning model, a prediction of whether the medical device is connected to the needleless connector.

17. The method of claim 11, wherein providing, with the at least one processor, the indication of whether the medical device is connected to the needleless connector includes:
generating, with the at least one processor, a message including at least: (i) the indication of whether the medical device is connected to the needleless connector, and (ii) an identifier of the medical device or of the needleless connector; and
communicating, with communication circuitry, the message to an external computing device.

18. The method of claim 11, wherein processing the set of images of the movable component of the needleless connector to determine whether the medical device is connected to the needleless connector includes:
comparing, with the at least one processor, pixels of an image of the set of images to pixels of another image of the set of images to determine a shift in a pixel pattern between the image and the another image;
converting, with the at least one processor, a shift in the pixel pattern between the image and the another image to a shift in x-coordinate distance of the movable component or a shift in y-coordinate distance of the movable component; and
determining, with the at least one processor, based on the shift in x-coordinate distance of the movable component or the shift in y-coordinate distance of the movable component, whether the medical device is connected to the needleless connector.

19. The method of claim 11, wherein processing the set of images of the movable component of the needleless connector to determine whether the medical device is connected to the needleless connector includes:
detecting, with the at least one processor, a frequency of one or more colors in one or more images of the set of images; and
determining, with the at least one processor, based on the frequency of the one or more colors in the one or more images of the set of images and one or more threshold frequencies, whether the medical device is connected to the needleless connector.

20. The method of claim 11, wherein the medical device includes a syringe that includes a Luer tip, wherein the moveable component has a first position when the Luer tip of the syringe is connected with the needleless connector and a second position when the Luer tip of the syringe is not connected with the needleless connector, and wherein processing the set of images of the movable component of the needleless connector to determine whether the medical device is connected to the needleless connector includes:
determining, based on the set of images, whether the moveable component is in the first position or the second position.

21. A computer program product comprising at least one non-transitory computer-readable medium including program instructions that, when executed by at least one processor, cause the at least one processor to:
receive a set of images of a movable component of a needleless connector;
process the set of images of the movable component of the needleless connector to determine whether a medical device is connected to the needleless connector; and
provide an indication of whether the medical device is connected to the needleless connector.
